# EUROPEAN PATENT SPECIFICATION

(11) **EP 2 189 146 B1**
(45) Date of publication and mention of the grant of the patent: **28.09.2011**
(21) Application number: 09382251.8
(22) Date of filing: 20.11.2009
(51) Int. Cl.: A61J 17/00

(54) **Geriatric pacifier**
Beruhigungsmittel für ältere Menschen
Tétine gériatrique

(30) Priority: 25.11.2008 ES 200803348
(43) Date of publication of application: 26.05.2010
(73) Proprietor: Paredes Sanchez, Esperanza del Carmen, 08304 Mataró-Barcelona (ES); Dominguez Adame, Antonio, 08304 Mataró-Barcelona (ES)
(72) Inventor: Paredes Sanchez, Esperanza del Carmen (75%), 08304, MATARÓ-BARCELONA (ES)
(74) Representative: Gallego Jiménez, José Fernando

(56) References cited:
- WO-A1-99/16405
- WO-A1-02/064079
- US-A- 5 891 165

## Description

### Object of the Invention

The present invention relates to a geriatric pacifier mainly intended for calming the mumbling and movement of the mouth caused mainly by anxiety.

### Background of the Invention

The prior art shows different types of pacifiers. See for example documents WO-A-02/064079 or WO-A-99/16405. The elderly and people of a considerably old age may present certain anxiety, which is usually reflected as mumbling and movement of mouth in an involuntary biting act. This causes them to experience a certain salivation or drooling and makes it possible for the teeth to cause an injury in the inner part of the lips.

Therefore, the technical problem set forth is the development of a pacifier for geriatric use which allows relieving the anxiety of the elderly.

### Description of the Invention

The geriatric pacifier object of this invention as claimed has technical particularities intended to allow the elderly user to relieve his or her anxiety by means of biting it during its use, preventing possible injuries from the effort he or she can make. The diagnosis of anxiety is associated with depression and factors such as cognitive deterioration, living alone, or certain stressing life events. This situation usually causes a tendency to suck on the lip and the tongue continuously. This problem is emphasized with the loss of teeth, therefore occasionally there ends up being a lack of stability in the mandibular joint, caused by repeated involuntary movements of the mandible, causing chattering between teeth and a sucking noise.

This geriatric pacifier is manufactured in latex or silicone, forming a body which comprises an internally secured thickened portion prolonged at the front part by a biting area with an arch-shaped and considerably planar configuration, this biting area being attached to an outwardly prolonged plate completely covering the corners of the mouth. The rear thickened portion has a rear concavity for the support and rest of the tongue and a front part with a rounded shape for its arrangement in the arch of the hard palate of the user's mouth. The biting area in turn has a dimensional width allowing it to be bitten by the front part of the mandible in approximately the width of the mouth. The user thus has a utensil on which he or she relieves his or her anxiety, avoiding injuring himself or herself and the unpleasant drooling effect, its constitution being ergonomic to enable a prolonged use.

It has been provided that the biting area has upper and lower surfaces with a certain transverse concavity to favor the correct seating of the teeth or gums, without them experiencing wear or injuries.

The ends of the biting area are concave and rounded to prevent injuries in the lips and to offer a more comfortable and hygienic use.

The thickened portion has at its lower part a V-shaped recess, below the concavity for the support of the tongue, for the arrangement of the frenum which everyone has. This allows the pacifier to adapt ergonomically to all the user population.

In an advanced embodiment, the outwardly prolonged plate has a planar configuration, arch-shaped in the horizontal plane, for its adaptation to the user's face. This plate has an inner rim for its entire perimeter and a plurality of through holes next to the inner part of this rim to prevent salivation by means of the sucking by the user.

It has been provided that the plate, the thickened portion and the biting area are formed integrally in a single part. Alternatively, the plate can be a removable part fixed on an intermediate support for its replacement, said intermediate support being large enough to prevent the person from swallowing the thickened portion in the event of an accidental detachment of the removable part.

In an alternative embodiment, the body of the pacifier has a groove from the outer face to the inner face in a front-rear direction, the purpose of which is the entrance/exit of an air flow sufficient for the correct lung function in those cases in which the user has certain respiratory problems, such as: glottal closure, cough, variable respiratory efforts, blocked nasal passages, mild bronchial obstruction, mild dyspnea, symptoms precipitated by exercise, cold, irritants, laughing, etc.

### Description of the Drawings

To complement the description which is being made and for the purpose of facilitating the understanding of the features of the invention, a set of drawings is attached to this specification, in which the following has been depicted with an illustrative and non-limiting character:

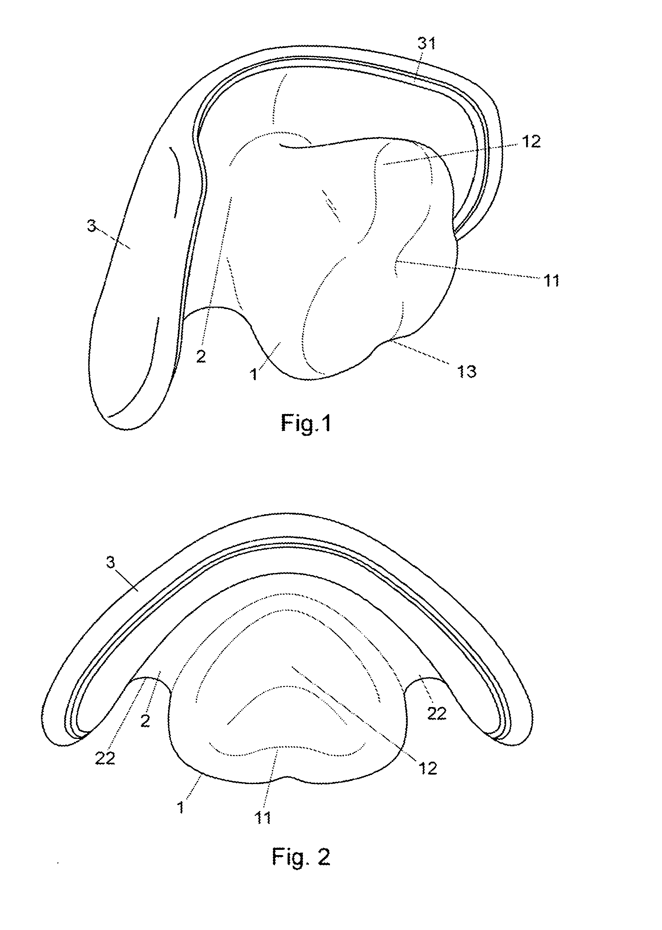

- Figure 1 shows a rear perspective view.
- Figure 2 shows a plan view.
- Figure 3 shows a rear view.
- Figure 4 shows a sectioned profile view in which the formation of the plate in a removable part and a fixing support is observed.
- Figure 5 shows respective front and rear views of an embodiment variant of this pacifier.

### Preferred Embodiment of the Invention

As can be observed in the referenced figures, the geriatric pacifier is formed as a single part, for example, in plastic or silicone, and comprises an internally secured thickened portion (1) which is prolonged at the front part by a biting area (2) with an arch-shaped and considerably planar configuration, this biting area (2) being attached to an outwardly prolonged plate (3) completely covering the corners of the mouth.

The rear thickened portion (1) has a rear concavity (11) for the support of the tongue and an arch-like upper part (12) for its arrangement in the arch of the hard palate of the user's mouth, a V-shaped recess (13) being located in the lower part of said thickened portion (1) for the arrangement of the frenum.

The biting area (2) has a dimensional width allowing it to be bitten by the front part of the mandible in approximately the width of the mouth to offer the greatest possible extension, but without causing discomfort during is use. The upper and lower surfaces (21) of the biting area (2) have a certain transverse concavity and preferably have a smooth texture, said biting area (2) being formed at one of its ends (22) in a concave and rounded manner to prevent injuries in the user's lips.

The outwardly prolonged plate (3) of the pacifier has an approximately planar configuration, arched in the horizontal plane for its adaptation to the user's face. This plate (3) has an inner rim (31) for its entire perimeter and a plurality of through holes (32) next to the inner part of this rim (31) to prevent salivation, promoting the flow of such saliva towards the mouth by sucking.

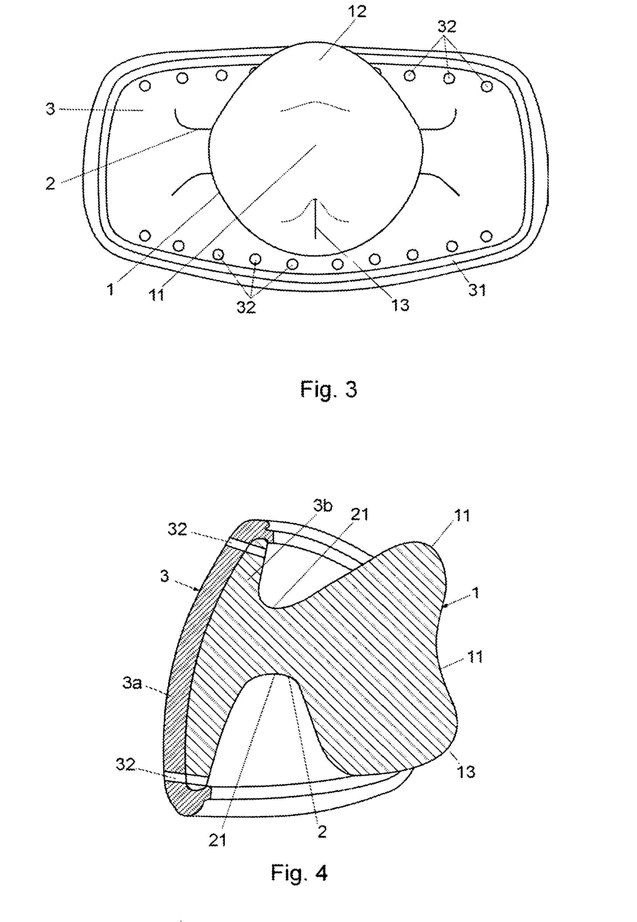

In an embodiment, depicted in Figure 4, the plate (3) is a removable part (3a) fixed on an intermediate support (3b) for its replacement, as an option to the formation in a single part.

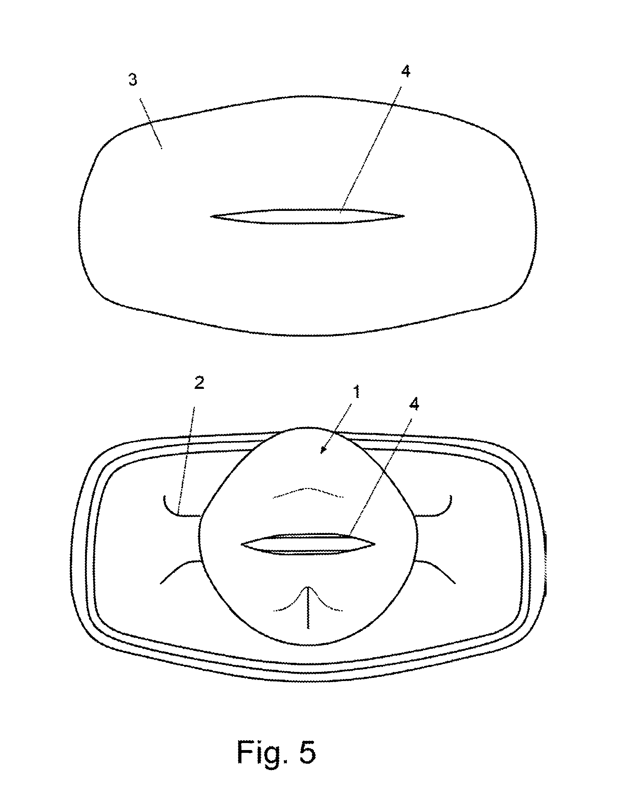

The embodiment depicted in Figure 5 shows a groove (4) existing in the body of the pacifier from the outer face to the inner face in a front-rear direction, the purpose of which is the entrance/exit of an air flow sufficient for the correct lung function in those cases in which the user has certain respiratory problems.

Having sufficiently described the nature of the invention, as well as a preferred embodiment, it is stated for the relevant purposes that the materials, shape, size and arrangement of the described elements can be modified, provided that this does not involve an alteration of the essential features of the invention which are claimed below.

## Claims

1. A geriatric pacifier comprising an internally secured thickened portion (1) having a rear concavity (11) for the support of the tongue and prolonged at the front part by a biting area (2) with a considerably planar configuration having a dimensional width allowing it to be bitten by the front part of the mandible in approximately the width of the mouth, this biting area (2) being attached to an outwardly prolonged plate (3) completely covering the corners of the mouth, **characterized in that** the biting area (2) has an arch-shaped configuration and the rear thickened portion (1) has an upper part (12) with a rounded shape for its arrangement in the arch of the hard palate of the user's mouth.

2. The pacifier according to claim 1, **characterized in that** the upper and lower surfaces (21) of the biting area have a certain transverse concavity.

3. The pacifier according to claim 1, **characterized in that** the ends (22) of the biting area (2) are concave and rounded to prevent injuries in the user's lips.

4. The pacifier according to claim 1, **characterized in that** the thickened portion (1) has at its lower part a V-shaped recess (13), below the concavity (11) for the support of the tongue, for the arrangement of the frenum of the tongue.

5. The pacifier according to claim 1, **characterized in that** the outwardly prolonged plate (3) has a planar configuration, arch-shaped in the horizontal plane, for its adaptation to the user's face.

6. The pacifier according to claim 5, **characterized in that** the plate (3) has an inner rim (31) for its entire perimeter and a plurality of through holes (32) next to the inner part of this rim (31) to prevent salivation.

7. The pacifier according to claim 1, **characterized in that** the plate (3) is a removable part (3a) fixed on an intermediate support (3b) for its replacement.

8. The pacifier according to claim 1, **characterized in that** the plate (3), the thickened portion (1) and the biting area (2) are integrally formed in a single part.

9. The pacifier according to claim 1, **characterized in that** it has a groove from the outer face to the inner face in a front-rear direction, the purpose of which is the entrance/exit of an air flow sufficient for the correct lung function of the user.

## Patentansprüche

1. Beruhigungsmittel für ältere Menschen, umfassend einen innen befestigten verdickten Abschnitt (1), der einen hinteren Hohlraum (11) zur Unterstützung der Zunge aufweist und an dem vorderen Teil durch einen Beißbereich (2) mit einer im Wesentlichen planaren Konfiguration verlängert ist, der eine derartige dimensionale Breite aufweist, dass durch den vorderen Teil des Kiefers in ungefähr der Breite des Mundes auf ihn gebissen werden kann, wobei dieser Beißbereich (2) an einer nach außen verlängerten Platte (3) angebracht ist, welche die Mundwinkel vollständig abdeckt, **dadurch gekennzeichnet, dass** der Beißbereich (2) eine bogenförmige Konfiguration aufweist und der hintere verdickte Abschnitt (1) einen oberen Teil (12) mit einer gerundeten Form aufweist, der in dem Bogen des Vordergaumens des Mundes des Benutzers angeordnet wird.

2. Beruhigungsmittel nach Anspruch 1, **dadurch gekennzeichnet, dass** die obere und die untere Oberfläche (21) des Beißbereichs einen quer verlaufenden Hohlraum aufweisen.

3. Beruhigungsmittel nach Anspruch 1, **dadurch gekennzeichnet, dass** die Enden (22) des Beißbereichs (2) konkav und gerundet sind, um Verletzungen an den Lippen des Benutzers zu verhindern.

4. Beruhigungsmittel nach Anspruch 1, **dadurch gekennzeichnet, dass** der verdickte Abschnitt (1) an seinem unteren Teil eine V-förmige Aussparung (13) unterhalb des Hohlraums (11) zur Unterstützung der Zunge und zur Anordnung des Frenums der Zunge aufweist.

5. Beruhigungsmittel nach Anspruch 1, **dadurch gekennzeichnet, dass** die nach außen verlängerte Platte (3) eine planare Konfiguration aufweist, die in horizontaler Ebene bogenförmig ist, damit sie sich an das Gesicht des Benutzers anpasst.

6. Beruhigungsmittel nach Anspruch 5, **dadurch gekennzeichnet, dass** die Platte (3) einen inneren Rand (31) entlang ihres gesamten Umfangs und mehrere Durchgangslöcher (32) benachbart des inneren Teils dieses Randes (31) aufweist, um Speichelfluss zu verhindern.

7. Beruhigungsmittel nach Anspruch 1, **dadurch gekennzeichnet, dass** die Platte (3) ein entfernbarer Teil (3a) ist, der an einer Zwischenstütze (3b) ist, um ersetzt werden zu können.

8. Beruhigungsmittel nach Anspruch 1, **dadurch gekennzeichnet, dass** die Platte (3), der verdickte Abschnitt (1) und der Beißbereich (2) einstückig als ein einziges Teil ausgebildet sind.

9. Beruhigungsmittel nach Anspruch 1, **dadurch gekennzeichnet, dass** es von der äußeren Fläche zu der inneren Fläche in einer Richtung von vorne nach hinten eine Rille aufweist, deren Zweck der Einlass/Auslass eines Luftstroms ist, der für die korrekte Lungenfunktion des Benutzers ausreichend ist.

## Revendications

1. Tétine gériatrique comprenant une portion épaissie internement connectée
(1) à une concavité arrière (11) pour le support de la langue et prolongée à la partie avant par une zone de morsure (2) avec une configuration considérablement planaire, ayant une largeur dimensionnelle qui permet sa morsure par la partie avant d'une mandibule ayant une largeur comparable à celle de la bouche, cette zone de morsure (2) étant connectée à une plaque prolongée vers l'extérieur (3) couvrant complètement les angles de la bouche, **caractérisée en ce que** la zone de morsure (2) présente une configuration en arcade et **en ce que** la portion épaissie arrière (1) a une partie supérieure (12) ayant une forme arrondie pour son introduction dans l'arc de la volute du palais de la bouche de l'utilisateur.

2. Tétine, conformément à la revendication 1, **caractérisée en ce que** les surfaces supérieure et inférieure (21) de la zone de morsure ont une certaine concavité transversale.

3. Tétine, conformément à la revendication 1, **caractérisée en ce que** les extrémités (22) de la zone de morsure (2) sont concaves et arrondies pour éviter de blesser les lèvres de l'utilisateur.

4. Tétine, conformément à la revendication 1, **caractérisée en ce que** la portion épaissie (1) présente à sa partie inférieure un retrait en forme de V (13), sous la concavité (11), pour le support de la langue, pour la disposition du frein de la langue.

5. Tétine, conformément à la revendication 1, **caractérisée en ce que** la plaque prolongée vers l'extérieur (3) présente une configuration planaire, en arcade sur la surface horizontale, pour permettre son adaptation au visage de l'utilisateur.

6. Tétine, conformément à la revendication 5, **caractérisée en ce que** la plaque (3) présente un côté intérieur (31) sur la totalité du périmètre et une pluralité d'orifices de passage (32) près de la partie intérieure de ce côté (31) pour éviter la salivation.

7. Tétine conformément à la revendication 1, **caractérisée en ce que** la plaque (3) est une partie amovible (3a) fixée à un support intermédiaire (3b) qui permet son remplacement.

8. Tétine conformément à la revendication 1, **caractérisée en ce que** la portion épaissie (1) et la zone de morsure (2) sont intégralement formées dans une seule pièce.

9. Tétine, conformément à la revendication 1, **caractérisée en ce qu'**elle présente une rainure de la partie extérieure à la partie intérieure en suivant une direction d'avant en arrière, dont l'objectif est l'entrée/ sortie du débit d'air suffisant pour la correcte fonction pulmonaire de l'utilisateur.
